# EUROPÄISCHE PATENTSCHRIFT

(11) **EP 2 395 965 B1**
(45) Veröffentlichungstag und Bekanntmachung des Hinweises auf die Patenterteilung: **04.04.2018**
(21) Anmeldenummer: 10705841.4
(22) Anmeldetag: 12.02.2010
(51) Int. Cl.: A61K 8/14, A61K 8/41, A61K 8/55, A61Q 19/00

(54) **KATIONISCHES TRÄGERSYSTEM AUS POSITIV GELADENEN LIPIDVESIKELN**
CATIONIC CARRIER SYSTEM COMPOSED OF POSITIVELY CHARGED LIPID VESICLES
SYSTÈME VECTEUR CATIONIQUE À BASE DE VÉSICULES LIPIDIQUES À CHARGE POSITIVE

(30) Priorität: 12.02.2009 DE 102009000824
(43) Veröffentlichungstag der Anmeldung: 21.12.2011
(73) Patentinhaber: Evonik Schlüchtern GmbH, 36381 Schlüchtern (DE)
(72) Erfinder: TEICHMÜLLER, Dirk, 63589 Linsengericht (DE); BEYER, Monika, 61130 Nidderau-Ostheim (DE)
(74) Vertreter: Andrae | Westendorp Patentanwälte Partnerschaft
(86) Internationale Anmeldenummer: PCT/EP2010/051773
(87) Internationale Veröffentlichungsnummer: WO 2010/092142

(56) Entgegenhaltungen:
- EP-A2- 1 060 732
- WO-A1-03/020037
- WO-A1-2010/052093
- DE-A1-102004 045 186
- US-A1- 2009 123 578
- Dirk Häfner: "Dissertation : Untersuchungen zu Wechselwirkungen zwischen flexiblen kationischen Lipidvesikeln und DNS sowie in vitro und in vivo Eigenschaften der daraus hergestellten Komplexe", 2002

## Beschreibung

Die vorliegende Erfindung betrifft kosmetische oder pharmazeutische Zusammensetzungen mit wenigstens einem Wirkstoff und mit einem Trägersystem für diesen wenigstens einen Wirkstoff, die Verwendung solcher Zusammensetzungen in kosmetischen und/oder pharmazeutischen Formulierungen sowie ein Verfahren zur Herstellung der Zusammensetzungen.

Viele Wirkstoffe weisen eine relativ geringe Stabilität auf und können auch schon durch an und für sich recht milde Umwelteinflüsse leicht inaktiviert werden. Beispielsweise wird Retinol in Gegenwart von Wasser bereits bei Raumtemperatur schnell inaktiviert.

Aber nicht nur die Stabilität des Retinols sondern auch die Menge des in die Zellen transportierten Retinols sind die Wirksamkeit beschränkende Faktoren, ein Phänomen, das gleichsam auch bei einer Vielzahl von anderen Wirkstoffen feststellbar ist.

Dokument DE 10 2004 045 186 A1 offenbart hydratisierte lamellare Phasen oder Liposomen, enthaltend ein Fettmonoamin oder ein kationisches Polymer, das die intrazelluläre Penetration begünstigt. Der vorliegenden Erfindung liegt daher die Aufgabe zugrunde, Mittel für eine verbesserte Stabilisierung und Freisetzung von Wirkstoffen zur Verfügung zu stellen. Insgesamt soll die Bioverfügbarkeit der Wirkstoffe gerade in den lebenden Hautzellen erhöht werden.

Diese Aufgabe wird erfindungsgemäß gelöst durch Zusammensetzungen , mit wenigstens einem Wirkstoff und mit einem Trägersystem für den wenigstens einen Wirkstoff, wobei das Trägersystem Lipidvesikel mit einer oder zwei Lipidmembran(en) umfasst, dadurch gekennzeichnet, dass der wenigstens eine Wirkstoff in den Lipidvesikeln enthalten ist und die Lipide, aus denen die Vesikel aufgebaut sind, unter Ceramiden, Phospholipiden, Glycosphingolipiden und/oder DiAcylglycosiden ausgewählt sind, wobei die Lipidvesikel eine positive Oberflächenladung aufweisen, die dadurch bewirkt wird, dass die Lipidvesikel in der/den Lipidmembran/en zusätzlich zu den Lipiden, aus denen die Vesikel aufgebaut sind, positiv geladene quartäre Ammoniumverbindungen als Ladungsgeber aufweisen, wobei diese Ladungsgeber aus Alkyl-Trimoniumsalzen der Formel 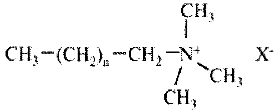 ausgewählt sind, wobei
n eine Zahl von 18 bis 28 und
X⁻ ein anorganisches oder organisches Anion ist.

Es konnte gezeigt werden, dass die erfindungsgemäße Zusammensetzung zu einer verbesserten Verfügbarkeit eines topisch applizierten Wirkstoffs in lebenden Hautzellen führt.

Die verbesserte Verfügbarkeit des applizierten Wirkstoffs basiert u.a. auf der positiven Ladung der Vesikeloberfläche. Die positive Ladung der Vesikeloberfläche führt zu einer verbesserten Anhaftung der Vesikel an der Oberfläche der lebenden Hautzellen und hierdurch dazu, daß der in den Vesikeln enhaltene Wirkstoff gezielt in lebenden Hautzellen zur Verfügung gestellt wird. Darüber hinaus führt die positive Ladung der Vesikel auch zu einer starken Anhaftung an der Haaroberfläche und zu einer Penetration der Vesikel in den Cortex der Haare.

Hierdurch wird die Menge an topisch appliziertem Wirkstoff, die in den Hautzellen oder in den Haaren bereitgestellt wird, signifikant erhöht, wobei diese Feststellungen in Bezug auf die vorliegende Erfindung jedoch nicht bindend sein sollen, und der Umfang der Erfindung soll durch diese Feststellungen auch nicht beschränkt werden.

Zusätzlich führt die erfindungsgemäße Zusammensetzung zu einer besseren Stabilisierung des topisch applizierten Wirkstoffs. Auch dies führt zu einer signifikanten Erhöhung der Menge an topisch appliziertem Wirkstoff, die in den Hautzellen oder in den Haaren bereitgestellt wird.

Die Erhöhung der Menge an topisch appliziertem Wirkstoff, die in den Hautzellen oder in den Haaren bereitgestellt wird, führt letztlich zu einer gesteigerten Wirksamkeit der Zusammensetzung bzw. der diese Zusammensetzung enthaltenden Formulierungen im Vergleich zu herkömmlichen Zusammensetzungen bzw. Formulierungen.

Die positive Ladung der Vesikeloberfläche wird erreicht, indem die Lipidvesikel zusätzlich zu den Lipiden, aus denen die Vesikel aufgebaut sind, positiv geladene Moleküle als Ladungsgeber umfassen.

Erfindungsgemäß handelt es sich bei den positiv geladenen Molekülen bzw. Ladungsgebern um Alkyl-Trimoniumsalze (bzw. Fettsäure-Trimoniumsalze) der Formel 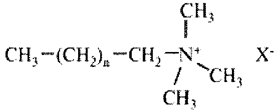 wobei n eine ganze Zahl von 18 bis 28 und X⁻ ein anorganisches oder organisches Anion ist.

Vorzugsweise ist X⁻ ein Halogenid-Ion oder das Anion einer organischen Säure, die ausgewählt ist unter einer kosmetisch oder pharmazeutisch verträglichen Carbonsäure oder Sulfonsäure. Besonders bevorzugt ist X⁻ Bromid, Chlorid, Fluorid, Iodid, Saccharinat, Tosylat oder Methosulfat.

Vorzugsweise ist n in der oben angegebenen Alkyl-Trimoniumsalzformel gleich 22. Inbesondere bevorzugt ist das Alkyl-Trimoniumsalz Behentrimoniumchlorid.

Vorzugweise werden die positiv geladenen Ladungsgeber in einer Menge von 0,01 bis 10 Gew.-%, bevorzugt 0,01 bis 2,0 Gew.-%, bezogen auf die gesamte Zusammensetzung eingesetzt.

Die Teilchengröße der erfindungsgemäßen Lipidvesikel ist vorzugsweise von 50 bis 1000 nm, 100 bis 400 nm, mehr bevorzugt 100 bis 350 nm, am meisten bevorzugt 100 bis 250 nm.

Die erfindungsgemäße Zusammensetzung kann Vesikel mit einer Lipidmembran (Nanosomen) oder zwei Lipidmembranen (Liposomen) umfassen. Dadurch wird eine Anpassung an verschiedene Verwendungen der Zusammensetzung ermöglicht.

Die Lipide sind unter Ceramiden, Phospholipiden, Glycosphingolipiden und/oder DiAcylglycosiden ausgewählt. Dazu gehören auch Sphingomyeline, Galactocerebroside und Glucocerebroside, Dihexoside, Tri-und Tetrahexoside sowie Ganglioside. Die Phospholipide, die bei der Zusammensetzung für die Bildung der Vesikel verwendet werden können, können unter allen kosmetisch oder pharmazeutisch verträglichen Phospholipiden ausgewählt werden, die in der Lage sind, in wässrigem Milieu Vesikel (Nanosomen oder Liposomen) zu bilden. Bevorzugte Phospholipide sind Lecithin, Phosphatidylcholin, Phosphatidylethanolamin und Phosphatidylserin.

Bei speziellen Ausführungsformen können auch Gemische der oben genannten Lipide eingesetzt werden. Der Anteil der Lipide bezogen auf die gesamte Zusammensetzung beträgt vorzugsweise 1 bis 20 Gew.-%, bevorzugt 1 bis 15 Gew.-%, besonders bevorzugt 4 bis 10 Gew.-%.

Als Wirkstoffe kommen bei der vorliegenden Erfindung uneingeschränkt alle physiologisch aktiven Wirkstoffe in Betracht, die verkapselt werden können. Hierbei kann es sich sowohl um lipophile als auch um hydrophile Substanzen handeln, die aus natürlichen Quellen isoliert oder chemisch oder biotechnologisch hergestellt wurden.

Beispiele für physiologisch aktive Wirkstoffe, die bei der vorliegenden Erfindung zur Anwendung kommen können, sind: natürliche Extrakte, pflanzliche Stoffe, Öle, Fette, Fettsäuren, Vitamine, Zucker, Proteine, Peptide, Aminosäuren, synthetische Wirkstoffe, anorganische Stoffe, leicht oxidierbare oder instabile Substanzen, kosmetische Wirkstoffe.

Bei einer bevorzugten Ausführungsform ist der Wirkstoff Retinol. Retinol, auch Vitamin A oder Vitamin A1 genannt, ist ein fettlösliches Molekül, welches an Prozessen beteiligt ist, die u.a. die Aufrechterhaltung des Sehvermögens, intakte Schleimhäute und eine intakte Haut sowie Nagel- und Haarwachstum bewirken.

Vorzugsweise beträgt der Anteil an Wirkstoff bei der vorliegenden Erfindung bezogen auf die gesamte Zusammensetzung 0,01 bis 40 Gew.-%.

In einer Ausführungsform der vorliegenden Erfindung ist der Wirkstoff in der/den Lipidmembran(en) enthalten, d.h. zwischen den Lipiden der Lipidmembran in die Lipidmembran eingebunden. Vorzugsweise beträgt der Anteil an Wirkstoff bei diesen Ausführungsformen bezogen auf die gesamte Zusammensetzung 0,01 bis 15 Gew.-%. Handelt es sich bei dem wenigstens eine Wirkstoff allerdings um einen lipophilen Wirkstoff, wie z.B. Retinol, beträgt der Anteil dieses Wirkstoff bezogen auf die gesamte Zusammensetzung vorzugsweise 0,01 bis 10 Gew.-%.

In einer anderen Ausführungsform ist der Wirkstoff von der/den Lipidmembran(en) umkapselt. Der Begriff "umkapselt" ist hier so zu verstehen, dass der Wirkstoff in einer Flüssigkeit, die sich in dem von einer Lipidmembran gebildeten Vesikelinnenraum befindet, gelöst vorliegt. Bei einer bevorzugten Ausführungsform liegt der Wirkstoff in einer im Vesikelinneren eingeschlossenen Ölphase gelöst vor. Vorzugsweise beträgt der Anteil an Wirkstoff bei diesen Ausführungsformen bezogen auf die gesamte Zusammensetzung 5 bis 40 Gew.-%.

Es hat sich gezeigt, dass Wirkstoffe, wenn sie im Vesikelinnenraum eingeschlossen vorliegen, bzw. wenn sie in der Lipidmembran eines Vesikels enthalten sind, stabilisiert werden. Der Begriff "stabilisiert" bezeichnet dabei, daß der umkapselte bzw. in der Lipidmembran vorliegende Wirkstoff im Vergleich zu frei vorliegendem Wirkstoff vor einer chemischen Veränderung, z.B. in der Gegenwart von Wasser, und damit auch vor einer Inaktivierung geschützt ist.

Erfindungsgemäß wird die Zusammensetzung in kosmetischen und/oder pharmazeutischen Formulierungen verwendet, wobei pharmazeutische Formulierungen solche sind, die unter das Arzneimittelrecht fallen. Dabei können der Zusammensetzung, die positiv geladene Lipidvesikel und wenigstens einen Wirkstoff umfasst, weitere Wirkstoffe hinzugefügt werden, die entweder in den Lipidvesikeln oder außerhalb der Lipidvesikel und in der übrigen Substanz bzw. Trägermatrix der Formulierung enthalten sind.

Die Formulierungen können alle Hilfs-und Zusatzstoffe, die üblicherweise bei kosmetischen oder pharmazeutischen Präparaten Anwendung finden, enthalten. Insbesondere fallen unter den Begriff "Hilfsstoff" im Zusammenhang mit der vorliegenden Erfindung solche Zusatzstoffe, die auf die physikalischen Eigenschaften der Vesikel und deren Stabilität einwirken und/oder der Konservierung der Zusammensetzung dienen. Beispiele für solche Hilfsstoffe sind Öle, Alkohole, Polyole, Antioxidantien, Gelbildner, Puffer, Konservierungsmittel, Bakterizide und Keimhemmer, Verdicker oder Komplexbildner.

Bevorzugt wird die erfindungsgemäße Zusammensetzung für kosmetische und/oder pharmazeutische Formulierungen verwendet, die zur topischen Applikation geeignet sind. Die erfindungsgemäße Zusammensetzung kann in allen für die topische Applikation geeigneten Formulierungen vorliegen, beispielsweise in Form eines Gels, einer Creme, einer Salbe, eines Sprays oder einer Lotion. Dazu kann die erfindungsgemäße Zusammensetzung in eine Trägermatrix eingearbeitet werden. Bei der Trägermatrix kann es sich um Gelformulierungen, Cremeformulierungen, Lotionen, Maskenanwendungen etc. handeln.

Für Anwendungen im Hautbereich wird die erfindungsgemäße Zusammensetzung vorzugsweise in einer Lotion, einer Creme, einer Salbe, einem Gel, einem wässrigen Fluid, einem Gesichtswasser, einem Sonnenprodukt oder einer Maske angewendet.

Für Anwendungen im Haarbereich wird die erfindungsgemäße Zusammensetzung vorzugsweise in einem Shampoo (vorzugsweise mit milden Tensiden), einer Spülung, einem Haargel, einem Conditioner, einem Hair Tonic, einem Haar-Styling-Produkt oder einem Haar-Pflege-Produkt angewendet.

Die Anwendungsbereiche der die erfindungsgemäße Zusammensetzung enthaltenden Formulierungen sind ohne Beschränkung hierauf beispielsweise Antifaltenwirkung, Anti Ageing-Wirkung, antioxidative Wirkung (Antioxidantien als Radikalfänger), Verstärkung der Barrierefunktion der Haut, befeuchtende und weichmachende Wirkung (Moisturizers und Emollients), Stimulierung der Kollagen Synthese, UV-Schutz, Anti-Akne-Wirkung, Anti-Cellulite-Wirkung, Stimulierung des Haarwachstums, Hemmung des Haarwachstums, Hautaufhellung, Hautbräunung und Entzündungshemmung.

Selbstverständlich handelt es sich bei allen Komponenten der erfindungsgemäßen Zusammensetzungen und Formulierungen um pharmazeutisch, kosmetisch oder dermatologisch verträgliche Stoffe. Ein Stoff ist im Sinne dieser Erfindung pharmazeutisch, kosmetisch oder dermatologisch verträglich, wenn er nicht toxisch ist und bei der Mehrzahl der potentiellen Anwender beispielsweise topisch anwendbar ist, ohne dass es bei dem Anwender spontan oder nach einer Weile zu einer ungewünschten physiologischen Reaktion, wie z.B. einer Rötung oder der Ausbildung eines Juckreizes kommt.

Bevorzugt werden die erfindungsgemäßen Zusammensetzungen nach einem Verfahren hergestellt, bei dem
a) ein Lipid, das unter Ceramiden, Phospholipiden, Glycosphingolipiden und/oder DiAcylglycosiden ausgewählt ist, in einem kurzkettigen aliphatischen Monoalkohol, bevorzugt Ethanol, gelöst wird, wobei eine Lipidlösung erhalten wird,
b) ein positiv geladenes Alkyl-Trimoniumsalz in der Lipidlösung aus a) gelöst wird,
c) wenigstens ein lipophiler Wirkstoff in die Lösung aus b) gegeben wird
d) die Lösung aus c) unter Rühren in eine Wasserphase gegeben wird, wobei eine Emulsion erhalten wird,
e) der pH-Wert der Emulsion durch Zugabe einer Base, bevorzugt Natronlauge, auf einen physiologischen pH-Wert von pH 6 bis 7 eingestellt wird, und
f) optional die Emulsion durch Hochdruckhomogenisation bei einem Druck von bis zu 1000 bar bis 1500 bar homogenisiert wird.

Unter einem kurzkettigen aliphatischen Monoalkohol ist dabei ein Monoalkohol mit einer Kettenlänge von C1 bis C4 zu verstehen.

Alternativ können erfindungsgemäße Zusammensetzungen nach einem Verfahren hergestellt werden, bei dem
a) ein Lipid, das unter Ceramiden, Phospholipiden, Glycosphingolipiden und/oder DiAcylglycosiden ausgewählt ist, in einem kurzkettigen aliphatischen Monoalkohol, bevorzugt Ethanol, gelöst wird, wobei eine Lipidlösung erhalten wird,
b) ein positiv geladenes Alkyl-Trimoniumsalz in der Lipidlösung von a) gelöst wird,
c) wenigstens ein hydrophiler Wirkstoff in eine Wasserphase gegeben wird
d) die Lösung aus b) unter Rühren in die Wasserphase aus c) gegeben wird, wobei eine Emulsion erhalten wird,
e) der pH-Wert der Emulsion durch Zugabe einer Base, bevorzugt Natronlauge, auf einen physiologischen pH-Wert von pH 6 bis 7 eingestellt wird und
f) optional die Emulsion durch Hochdruckhomogenisation bei einem Druck von bis zu 1000 bar bis 1500 bar homogenisiert wird.

Bei den erfindungsgemäßen Zusammensetzungen, die sowohl einen lipophilen als auch einen hydrophilen Wirkstoff enthalten, ist eine Kombination der beiden oben beschriebenen Verfahren besonders bevorzugt.

Für Zwecke der ursprünglichen Offenbarung wird darauf hingewiesen, dass sämtliche Merkmale, wie sie sich aus der vorliegenden Beschreibung und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder chemische, physikalisch-chemische, kosmetische, pharmakologische oder dermatologische Gegebenheiten derartige Kombinationen unmöglich oder sinnlos machen. Auf die umfassende, explizite Darstellung sämtlicher denkbaren Merkmalskombinationen wird hier nur der Kürze und der Lesbarkeit der Beschreibung wegen verzichtet.

Des Weiteren wird darauf hingewiesen, dass es für den Fachmann selbstverständlich ist, dass die nachfolgenden Ausführungsbeispiele lediglich dazu dienen, zwei mögliche Ausführungsformen der vorliegenden Erfindung beispielhaft anzugeben. Der Fachmann wird daher ohne weiteres verstehen, dass darüber hinaus auch alle anderen Ausführungsformen, die die in den Ansprüchen genannten erfindungsgemäßen Merkmale oder Merkmalskombinationen aufweisen, innerhalb des Schutzumfangs der Erfindung liegen. Auf die umfassende, explizite Darstellung sämtlicher denkbarer Ausführungsformen innerhalb der beanspruchten Erfindung wird hier nur der Kürze und der Lesbarkeit der Beschreibung wegen verzichtet.

### Ausführungsbeispiel1 (Wirkstoff in der Lipidmembran)

Ausführungsbeispiel 1 betrifft Lipidvesikel mit der folgenden Zusammensetzung:
10,00 Gew.-% Phospholipide (Lecithin)
12,00 Gew.-% Ethanol
4,00 Gew.-% Behentrimoniumchlorid
1,00 Gew.-% Wirkstoff (Retinol)
Ad 100 Gew.-% Wasser, VE

Um die Lipidvesikel gemäß Ausführungsbeispiel 1 herzustellen, wurde das Lecithin in Ethanol vollständig solubilisiert. In diese Lösung wurden Behentrimoniumchlorid und eine Vitaminzubereitung mit 50 Gew.-% Retinol unter Rühren eingearbeitet und vollständig gelöst. Nach einem Homogenisationsverfahren wurde diese Lipidphase in Wasser eingearbeitet.

Die Teilchengröße der nach diesem Verfahren hergestellten kationischen Lipidvesikel wurde mit Hilfe der Photonenkorrelationsspektroskopie (PCS, Zetamaster S, Malvern Instruments Ud., UK) kontrolliert, wobei der Herstellungsprozess nach Erreichen einer mittleren Teilchengröße mit einem Durchmesser von etwa 100 bis 350 nm beendet wurde.

### Ausführungsbeispiel 2 (Wirkstoff in Lösung im Vesikelinnenraum)

Ausführungsbeispiel 2 betrifft Lipidvesikel mit der folgenden Zusammensetzung:
7,00 Gew.-% Phospholipide (Lecithin)
12,00 Gew.-% Ethanol
3,00 Gew.-% Behentrimoniumchlorid
5,00 Gew.-% Wirkstoff (Retinol)
12,00 Gew.-% Sojaöl
Ad 100 Gew.-% Wasser, VE

Um die Lipidvesikel gemäß Ausführungsbeispiel 2 herzustellen, wurde das Lecithin in Ethanol vollständig solubilisiert. In diese Lösung wurden nacheinander Behentrimoniumchlorid sowie eine Vitaminzubereitung aus Retinol und Sojaöl unter Rühren eingearbeitet. In diese Lipidphase wurde schließlich die angegebene Wassermenge homogen eingearbeitet und dabei mit der Lipidphase gemischt. Anschließend wurde diese Emulsion mit Hilfe der Hochdruckhomogenisation homogenisiert.

Die Teilchengröße der nach diesem Verfahren hergestellten kationischen Lipidvesikel wurde mit Hilfe der Photonenkorrelationsspektroskopie (PCS, Zetamaster S, Malvern Instruments Ud., UK) kontrolliert, wobei der Herstellungsprozess nach Erreichen einer mittleren Teilchengröße mit einem Durchmesser von etwa 100 bis 400 nm beendet wurde.

## Patentansprüche

1. Kosmetische oder pharmazeutische Zusammensetzung mit wenigstens einem Wirkstoff und mit einem Trägersystem für den wenigstens einen Wirkstoff, wobei das Trägersystem Lipidvesikel mit einer oder zwei Lipidmembran(en) umfasst, **dadurch gekennzeichnet, dass** der wenigstens eine Wirkstoff in den Lipidvesikeln enthalten ist und die Lipide, aus denen die Vesikel aufgebaut sind, unter Ceramiden, Phospholipiden, Glycosphingolipiden und/oder Di-Acylglycosiden ausgewählt sind, wobei die Lipidvesikel eine positive Oberflächenladung aufweisen, die dadurch bewirkt wird, dass die Lipidvesikel in der/den Lipidmembran/en zusätzlich zu den Lipiden, aus denen die Vesikel aufgebaut sind, positiv geladene Moleküle als Ladungsgeber aufweisen, wobei diese Ladungsgeber aus Alkyl-Trimoniumsalzen der Formel 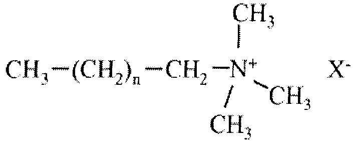 ausgewählt sind, wobei
n eine Zahl von 18 bis 28 und
X⁻ ein anorganisches oder organisches Anion ist.

2. Zusammensetzung gemäß Anspruch 1, **dadurch gekennzeichnet, dass** X- Bromid, Chlorid, Fluorid, Iodid, Saccharinat, Tosylat oder Methosulfat ist.

3. Zusammensetzung gemäß einem der Ansprüche 1 oder 2, **dadurch gekennzeichnet, dass** der Anteil der Ladungsgeber bezogen auf die gesamte Zusammensetzung 0,01 bis 10 Gew.-% beträgt.

4. Zusammensetzung gemäß einem der vorangehenden Ansprüche, **dadurch gekennzeichnet, dass** die Lipidvesikel eine Teilchengröße von 50 bis 1000 nm haben.

5. Zusammensetzung gemäß einem der vorangehenden Ansprüche, **dadurch gekennzeichnet, dass** der Anteil der Lipide bezogen auf die gesamte Zusammensetzung 1 bis 20 Gew.-% beträgt.

6. Zusammensetzung gemäß einem der vorangehenden Ansprüche, **dadurch gekennzeichnet, dass** der wenigstens eine Wirkstoff ausgewählt ist unter lipophilen und hydrophilen physiologisch aktiven Substanzen, die aus natürlichen Quellen isoliert oder chemisch oder biotechnologisch hergestellt wurden, oder Kombinationen davon.

7. Zusammensetzung gemäß einem der vorangehenden Ansprüche, **dadurch gekennzeichnet, dass** der Anteil des wenigstens einen Wirkstoffs bezogen auf die gesamte Zusammensetzung 0,01 bis 40 Gew.-% beträgt.

8. Zusammensetzung gemäß einem der Ansprüche 1 bis 7, **dadurch gekennzeichnet, dass** sie zusätzlich einen oder mehrere Hilfsstoffe umfasst.

9. Verwendung einer Zusammensetzung gemäß einem der Ansprüche 1 bis 8 zur Herstellung einer kosmetischen und/oder pharmazeutischen Formulierung.

10. Kosmetische und/oder pharmazeutische Formulierung, die eine Zusammensetzung gemäß einem der Ansprüche 1 bis 8 enthält.

11. Verwendung gemäß Anspruch 9 oder Formulierung gemäß Anspruch 10, **dadurch gekennzeichnet, dass** die Formulierung für die topische Applikation ist.

12. Verfahren zur Herstellung von Zusammensetzungen gemäß einem der Ansprüche 1 bis 8, **dadurch gekennzeichnet, dass**
a) ein Lipid, das unter Ceramiden, Phospholipiden, Glycosphingolipiden und/oder Di-Acylglycosiden ausgewählt ist, in einem kurzkettigen aliphatischen Monoalkohol mit einer Kettenlänge von C1 bis C4 gelöst wird, wobei eine Lipidlösung erhalten wird,
b) ein positiv geladenes Alkyl-Trimoniumsalz, in der Lipidlösung von a) gelöst wird,
c) wenigstens ein lipophiler Wirkstoff in die Lösung aus b) gegeben wird,
d) die Lösung aus c) unter Rühren in eine Wasserphase gegeben wird, wobei eine Emulsion erhalten wird,
e) der pH-Wert der Emulsion durch Zugabe einer Base auf einen physiologischen pH-Wert von pH 6 bis 7 eingestellt wird, und
f) optional die Emulsion durch Hochdruckhomogenisation bei einem Druck von bis zu 1000 bar bis 1500 bar homogenisiert wird.

13. Verfahren zur Herstellung von Zusammensetzungen gemäß einem der Ansprüche 1 bis 8, **dadurch gekennzeichnet, dass**
a) ein Lipid, das unter Ceramiden, Phospholipiden, Glycosphingolipiden und/oder Di-Acylglycosiden ausgewählt ist, in einem kurzkettigen aliphatischen Monoalkohol mit einer Kettenlänge von C1 bis C4 gelöst wird, wobei eine Lipidlösung erhalten wird,
b) ein positiv geladenes Alkyl-Trimoniumsalz, in der Lipidlösung von a) gelöst wird,
c) wenigstens ein hydrophiler Wirkstoff in eine Wasserphase gegeben wird,
d) die Lösung aus b) unter Rühren in die Wasserphase aus c) gegeben wird, wobei eine Emulsion erhalten wird,
e) der pH-Wert der Emulsion durch Zugabe einer Base auf einen physiologischen pH-Wert von pH 6 bis 7 eingestellt wird, und
f) optional die Emulsion durch Hochdruckhomogenisation bei einem Druck von bis zu 1000 bar bis 1500 bar homogenisiert wird.

## Claims

1. Cosmetic or pharmaceutical preparation having at least one active ingredient and having a carrier system for the at least one active ingredient, wherein the carrier system comprises lipid vesicles with one or two lipid membrane(s), **characterized in that** the at least one active ingredient is present in the lipid vesicles and the lipids, of which the vesicles are composed, are selected from ceramides, phospholipids, glycosphingolipids and/or diacyl glycosides, wherein the lipid vesicles have a positively-charged surface, which thereby causes the lipid vesicles in the lipid membrane(s) in addition to the lipids, of which the vesicles are composed, to have positively-charged molecules as charge donors, wherein these charge donors are selected from alkyltrimonium salts of the formula 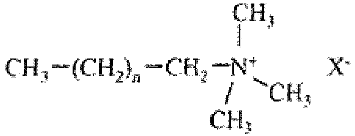 wherein
n is a number from 18 to 28 and
X⁻ is an inorganic or organic anion.

2. Composition according to Claim 1, **characterized in that** X⁻ is bromide, chloride, fluoride, iodide, saccharinate, tosylate or methosulfate.

3. Composition according to either of Claims 1 or 2, **characterized in that** the proportion of charge donors, based on the total composition, is 0.01 to 10% by weight.

4. Composition according to any of the preceding claims, **characterized in that** the lipid vesicles have a particle size of 50 to 1000 nm.

5. Composition according to any of the preceding claims, **characterized in that** the proportion of lipids, based on the total composition, is 1 to 20% by weight.

6. Composition according to any of the preceding claims, **characterized in that** the at least one active ingredient is selected from lipophilic and hydrophilic physiologically active substances which have been isolated from natural sources or have been produced chemically or biotechnologically, or combinations thereof.

7. Composition according to any of the preceding claims, **characterized in that** the proportion of the at least one active ingredient, based on the total composition, is 0.01 to 40% by weight.

8. Composition according to any of Claims 1 to 7, **characterized in that** said composition additionally comprises one or more auxiliaries.

9. Use of a composition according to any of Claims 1 to 8 for preparing a cosmetic and/or pharmaceutical formulation.

10. Cosmetic and/or pharmaceutical formulation, comprising a composition according to any of Claims 1 to 8.

11. Use according to Claim 9 or formulation according to Claim 10, **characterized in that** the formulation is for topical application.

12. Method for preparing compositions according to any of Claims 1 to 8, **characterized in that**
a) a lipid, selected from ceramides, phospholipids, glycosphingolipids and/or diacyl glycosides, is dissolved in a short-chain aliphatic monoalcohol having a chain length of C1 to C4, wherein a lipid solution is obtained,
b) a positively-charged alkyltrimonium salt is dissolved in the lipid solution from a),
c) at least one lipophilic active ingredient is added to the solution from b),
d) the solution from c) is added with stirring to a water phase, wherein an emulsion is obtained,
e) the pH of the emulsion is adjusted to a physiological pH of pH 6 to 7 by adding a base, and
f) optionally the emulsion is homogenized by high pressure homogenization at a pressure of up to 1000 bar to 1500 bar.

13. Method for preparing compositions according to any of Claims 1 to 8, **characterized in that**
a) a lipid, selected from ceramides, phospholipids, glycosphingolipids and/or diacyl glycosides, is dissolved in a short-chain aliphatic monoalcohol having a chain length of C1 to C4, wherein a lipid solution is obtained,
b) a positively-charged alkyltrimonium salt is dissolved in the lipid solution from a),
c) at least one hydrophilic active ingredient is added to a water phase,
d) the solution from b) is added with stirring to the water phase from c), wherein an emulsion is obtained,
e) the pH of the emulsion is adjusted to a physiological pH of pH 6 to 7 by adding a base, and
f) optionally the emulsion is homogenized by high pressure homogenization at a pressure of up to 1000 bar to 1500 bar.

## Revendications

1. Composition cosmétique ou pharmaceutique contenant au moins un agent actif et contenant un système vecteur pour ledit au moins un agent actif, le système vecteur comprenant des vésicules lipidiques contenant une ou deux membranes lipidiques, **caractérisée en ce que** ledit au moins un agent actif est contenu dans les vésicules lipidiques et les lipides à partir desquels les vésicules sont formées sont choisis parmi les céramides, les phospholipides, les glycosphingolipides et/ou les diacylglycosides, les vésicules lipidiques présentant une charge de surface positive qui est obtenue **en ce que** les vésicules lipidiques comprennent dans la/les membranes lipidiques en plus des lipides à partir desquels les vésicules sont formées des molécules chargées positivement en tant que donneurs de charges, ces donneurs de charges étant choisis parmi les sels d'alkyltrimonium de formule 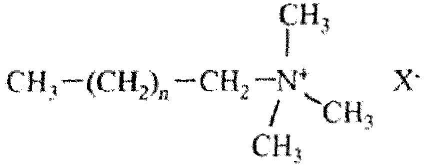 dans laquelle
n est un nombre de 18 à 28, et
X⁻ est un anion inorganique ou organique.

2. Composition selon la revendication 1, **caractérisée en ce que** X⁻ est un bromure, chlorure, fluorure, iodure, saccharinate, tosylate ou méthosulfate.

3. Composition selon l'une quelconque des revendications 1 ou 2, **caractérisée en ce que** la proportion des donneurs de charges par rapport à la composition totale est de 0,01 à 10 % en poids.

4. Composition selon l'une quelconque des revendications précédentes, **caractérisée en ce que** les vésicules lipidiques ont une taille de particule de 50 à 1 000 nm.

5. Composition selon l'une quelconque des revendications précédentes, **caractérisée en ce que** la proportion des lipides par rapport à la composition totale est de 1 à 20 % en poids.

6. Composition selon l'une quelconque des revendications précédentes, **caractérisée en ce que** ledit au moins un agent actif est choisi parmi les substances physiologiquement actives lipophiles et hydrophiles, qui ont été isolées à partir de sources naturelles ou fabriquées par voie chimique ou biotechnologique, ou leurs combinaisons.

7. Composition selon l'une quelconque des revendications précédentes, **caractérisée en ce que** la proportion dudit au moins un agent actif par rapport à la composition totale est de 0,01 à 40 % en poids.

8. Composition selon l'une quelconque des revendications 1 à 7, **caractérisée en ce qu'**elle comprend en outre un ou plusieurs adjuvants.

9. Utilisation d'une composition selon l'une quelconque des revendications 1 à 8 pour la fabrication d'une formulation cosmétique et/ou pharmaceutique.

10. Formulation cosmétique et/ou pharmaceutique, qui contient une composition selon l'une quelconque des revendications 1 à 8.

11. Utilisation selon la revendication 9 ou formulation selon la revendication 10, **caractérisée en ce que** la formulation est pour l'application topique.

12. Procédé de fabrication de compositions selon l'une quelconque des revendications 1 à 8, **caractérisé en ce que**
a) un lipide, qui est choisi parmi les céramides, les phospholipides, les glycosphingolipides et/ou les diacylglycosides, est dissous dans un monoalcool aliphatique à chaîne courte ayant une longueur de chaîne de C1 à C4, une solution de lipides étant obtenue,
b) un sel d'alkyl-trimonium chargé positivement est dissous dans la solution de lipides de a),
c) au moins un agent actif lipophile est introduit dans la solution de b),
d) la solution de c) est introduite sous agitation dans une phase aqueuse, une émulsion étant obtenue,
e) le pH de l'émulsion est ajusté par ajout d'une base à un pH physiologique de pH 6 à 7, et
f) l'émulsion est éventuellement homogénéisée par homogénéisation à pression élevée à une pression de jusqu'à 1 000 bar à 1 500 bar.

13. Procédé de fabrication de compositions selon l'une quelconque des revendications 1 à 8, **caractérisé en ce que**
a) un lipide, qui est choisi parmi les céramides, les phospholipides, les glycosphingolipides et/ou les diacylglycosides, est dissous dans un monoalcool aliphatique à chaîne courte ayant une longueur de chaîne de C1 à C4, une solution de lipides étant obtenue,
b) un sel d'alkyl-trimonium chargé positivement est dissous dans la solution de lipides de a),
c) au moins un agent actif hydrophile est introduit dans une phase aqueuse,
d) la solution de b) est introduite sous agitation dans la phase aqueuse de c), une émulsion étant obtenue,
e) le pH de l'émulsion est ajusté par ajout d'une base à un pH physiologique de pH 6 à 7, et
f) l'émulsion est éventuellement homogénéisée par homogénéisation à pression élevée à une pression de jusqu'à 1 000 bar à 1 500 bar.
